Europäisches Patentamt

European Patent Office

Office européen des brevets

(11) Publication number: **0 048 974**
**B1**

(12) **EUROPEAN PATENT SPECIFICATION**

(45) Date of publication of patent specification: **04.12.85**

(21) Application number: **81107634.8**

(22) Date of filing: **25.09.81**

(51) Int. Cl.⁴: **C 07 C 59/105,** C 07 C 51/235
// B01J19/26, B01J23/44

(54) Process for producing saccharide oxides.

(30) Priority: **29.09.80 JP 135484/80**

(43) Date of publication of application:
**07.04.82 Bulletin 82/14**

(45) Publication of the grant of the patent:
**04.12.85 Bulletin 85/49**

(84) Designated Contracting States:
**DE FR GB IT NL**

(56) References cited:
EP-A-0 000 902
DE-B-1 037 441
FR-A-2 334 409
FR-A-2 364 882
FR-A-2 366 868
GB-A-1 208 101
US-A-3 595 909

(73) Proprietor: **KAO SOAP COMPANY, LIMITED**
**Nihonbashi-Kayabacho 1-chome Chuo-ku**
**Tokyo (JP)**
(73) Proprietor: **Kawaken Fine Chemicals Co., Ltd.**
**6-6, Nihonbashi-Kobunacho Chuo-ku**
**Tokyo (JP)**

(72) Inventor: **Nakayama, Mutsuo**
**800-67, Kimiidera**
**Wakayama-shi (JP)**
Inventor: **Kimura, Akio**
**748.4 Sakata**
**Wakayama-shi (JP)**
Inventor: **Eguchi, Hiroshi**
**698-53, Musota**
**Wakayama-shi (JP)**
Inventor: **Matsui, Tadashi**
**283-43, Hatake, Iwadecho**
**Nagagun Wakayama (JP)**

(74) Representative: **Dickel, Klaus, Dipl.-Ing. et al**
**Julius-Kreis-Strasse 33**
**D-8000 München 60 (DE)**

Courier Press, Leamington Spa, England.

**Description**

The invention relates to a process for oxidizing a water-soluble saccharide to obtain the corresponding carboxylic acid derivative. More particularly the invention relates to a process for oxidizing a water-soluble saccharide to obtain the corresponding carboxylic acid derivative, which comprises: in a reaction vessel, contacting a gas containing molecular oxygen with a bath of a liquid reaction mixture of an aqueous solution of said saccharide having suspended therein particles of a palladium catalyst supported on carbon, at a temperature in the range of 30° to 60°C and a pH maintained in the range of 8 to 11, whereby to obtain said carboxylic acid derivative.

Saccharide oxides such as gluconic acid and salts thereof have been used for various purposes such as detergents for metal and glass surfaces, concrete additives, medicines and food additives. Currently, gluconic acid is produced on a commercial scale by the fermentation process wherein glucose is fermented using a microorganism. However, the microbiological fermentation process has many defects such as poor production efficiency, difficulty of the separation of microbial bodies, difficulty of the control of by-products and difficulty of the treatment of waste water. It is considered, under the circumstances as above, that the production process of saccharide oxides by the catalytic oxidation reaction carried out in the presence of a catalyst will be a most effective process in future. However, at present, the oxidation reaction cannot be carried out efficiently and satisfactory results are not always obtained by this process. Processes for the production of saccharide oxides such as gluconic acid by oxidizing saccharides such as glucose with an oxygen-containing gas such as air in the presence of a catalyst comprising a noble metal such as platinum or palladium carried on active carbon have been known from, for example, the specifications of US Patent No. 3,595,909 and West Germany Patent No. 1,037,441. However, fully satisfactory results cannot be obtained by merely mixing a saccharide with a catalyst while an oxygen-containing gas is introduced therein in an ordinary manner according to those processes.

If gluconic acid is to be obtained selectively from glucose yield of the intended product is impractically too small. If the conditions are made severer for the purpose of increasing the yield, the resulting product is colored and decomposition products are also formed to make the selective production of gluconic acid impossible.

Various gas-liquid contact reaction devices have been proposed. Differences among them are, however, insignificant when the ordinary reactions are carried out therein except for a little difference in the reaction velocity, though they each have merits and demerits.

After intensive investigations made for the purpose of finding a process for obtaining saccharide oxides which are substantially not colored in a short time with improved selectivity and yield by directly oxidizing saccharides with molecular oxygen according to gas-liquid contact reaction, the inventors have found that unexpected, remarkable effects can be obtained by employing a specific gas-liquid contacting process. The present invention has been completed on the basis of this finding.

According to the invention the above-mentioned process is characterized in continuously withdrawing a portion of said reaction mixture from said reaction vessel, feeding same through a pipe located outside of said vessel by means of a circulating pump, then feeding the reaction mixture from said pipe into and through a mixing injector, inducing a flow of said gas from a source of supply thereof into said mixing injector caused by the velocity of flow of said reaction mixture through said mixing injector and therein mixing said gas with said reaction mixture flowing through said mixing injector, and then feeding the mixture of said gas and said reaction mixture discharged from said mixing injector into said bath of said reaction mixture in said reaction vessel below the upper surface thereof, and wherein the ratio V2/V1 is in the range of from 0,1 to 10,0 and the ratio V3/V2 is in the range of 0,1 to 100.0, wherein V1 is the volume, in liters, of said bath in said vessel, V2 is the volumetric rate of flow, in liters per minute, of the reaction mixture circulating through said pipe and V3 is the volume, in liters per minute, of oxygen mixed with said reaction mixtures in said mixing injector.

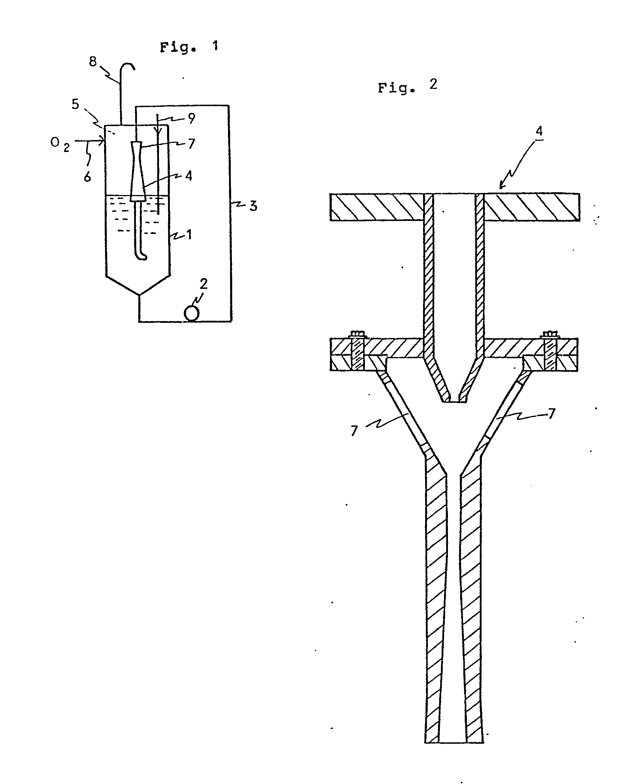

The present invention will be illustrated in more detail. As shown in Figure 1, the process for producing saccharide oxides of the present invention is characterized by the following points: In a reaction vessel 1, an end of a circulating pipe 3 through which the above aqueous suspension is passed is connected with an injector 4 of such a type that a gas is sucked and mixed therein as the circulating liquid is injected out. An oxygen-containing gas such as air or oxygen per se introduced in the reaction vessel 1 by the driving force of the circulating liquid is sucked through a gas inlet 7. The oxygen-containing gas is forcedly stirred in and mixed with the aqueous saccharide solution in which palladium carbon catalyst has been suspended, thereby carrying out the oxidation reaction.

According to the process of the present invention, it is made possible to produce saccharide oxides by the direct oxidation of saccharides such as glucose with a high efficiency which efficiency makes it possible to carry out the process on an industrial scale. Thus, according to this process, saccharide oxides of a high quality can be obtained at a high reaction velocity with a high selectivity. The saccharide oxides cannot be produced in a fully satisfactory manner when a reaction device generally used for the oxidation reaction such as stirring type reaction device is used, since in such a device, the reaction velocity is low, reaction selecitivity is poor and qualities such as hue of the product are poor.

The injector used in the present invention is of such a type that as the driving liquid is injected out, a surrounding gas is sucked therein. An embodiment of the injector is shown in Fig. 2. The shape and structure of the injector may be determined according to operation conditions by a method shown, in for example, "Kagaku Kogaku Binran (Handbook of Chemical Engineering)" edited by Kagaku Kogaku Kyokai. The amounts of the circulating liquid and oxygen-containing gas are preferably as large as possible for attaining the object of the present invention. The lowest limit of the amounts must be provided. The amounts of them will be shown as follows:

$V_1$: Amount of the suspension in the reaction vessel (liters),

$V_2$: Amount of the circulating liquid per minute (liters), and

$V_3$: Amount of oxygen gas sucked per minute (liters).

Ratio of $V_2/V_1$ should be at least 0.1. The operation conditions are preferably controlled so as to attain the ratio $V_2/V_1$ of 0.1—10. According to the process of the present invention, the gas-liquid contact reaction of the aqueous saccharide solution with oxygen is carried out in the reaction vessel. Further, the gas-liquid contact reaction is remarkably accelerated by the vigorous agitation in the injector. Therefore, it is necessary to pass as large as possible quantity of the liquid through the injector. If the ratio is too low, a long period of time is required for completing the reaction. Since the suspension of the solid catalyst (palladium carbon) is used in the reaction according to the present invention, stagnating parts in which the flow becomes motionless are formed unavoidably to precipitate and to accumulate the palladium carbon catalyst therein, thereby making the effective use of the catalyst impossible in the conventional reaction devices of stirring type. However, according to the process of the present invention, such a phenomenon as above does not occur, since the large quantity of the liquid in the reaction vessel is wholly circulated. The present invention is thus characterized in that the reaction is always carried out under the most suitable conditions. If $V_2/V_1$ ratio is less than 0.1, a long period of time is required for completing the reaction and the dispersion of the catalyst becomes uneven to make it impossible to attain the above described objects of the present invention. Taking also the economical advantage into consideration, the most practical, preferred operation conditions comprise a $V_2/V_1$ ratio of 0.1—10. As for the amount of the oxygen-containing gas to be sucked in, the larger, the better in principle. Namely, if value of $V_2$ is constant, the higher the $V_3/V_2$ ratio, the better. For increasing the $V_3/V_2$ ratio, there may be employed, for example, a method wherein the circulating liquid is introduced under pressure into the injector and sprayed. By this method, the gas sucked in can be increased remarkably to yield a $V_3/V_2$ ratio of as high as 100 or higher.

If the value of $V_3$ is extremely low, the oxygen supply into the reaction system becomes insufficient and an enough reaction velocity cannot be attained. Therefore, it is desirable to use an injector having a sucking capacity under atmospheric pressure of $V_3/V_2$ of 0.1—100 (oxygen).

Practically, a desired amount of the oxygen-containing gas can be sucked in by selecting an injector having a capacity suitable for attaining the purpose. In case capacity of an injector is insufficient, two or more injectors may be arranged in a row.

As the saccharides used in the present invention, there may be mentioned pentoses such as arabinose, xylose and ribose, hexoses such as glucose, mannose and galactose and other monosaccharides as well as polysaccharides obtained by the dehydration condensation of two or more molecules of those monosaccharides. The saccharides used in the present invention are soluble in water and they are subjected to the reaction in the form of an aqueous solution having a concentration of 5—50 wt.%. The catalyst used in the present invention is palladium carbon catalyst comprising palladium carried on active carbon. They can be prepared by known methods disclosed in, for example, the specifications of Japanese Patent Publication No. 7620/1958 and U.S. Patent No. 2,857,337. In carrying out the reaction according to the present invention, the palladium carbon catalyst is used in an amount of 0.005—0.5 wt.% (as palladium metal) based on the saccharide. As the oxygen-containing gas used as the oxidizing agent, there may be used oxygen, air. etc.

The oxidation reaction can be carried out at a temperature of 30—60°C under atmospheric pressure. If the reaction is carried out under an elevated pressure of up to about 10 atms, the reaction velocity can further be increased. An alkali such as an aqueous sodium hydroxide solution is added to the reaction mixture as occasion demands to maintain the mixture at pH 8—11, since the pH value lowers as the reaction proceeds.

It has been proved that according to the process of the present invention, the oxidation can be completed in a shorter time than that required in the known, conventional processes to obtain the saccharide oxides having an excellent hue with the formation of by-products in only a smaller amount. Those excellent effects could not be expected from the conventional processes. In addition, the process of the present invention wherein the device used is free of a gas inlet unlike the devices used in the conventional processes has a practical merit that the operation is easy, since it is free from the clogging of the inlet with the catalyst.

In the process of the present invention, the injector may be placed either at an end of the circulating pipe in the reaction vessel or in a midway of the circulating line to suck air in or to feed oxygen. In case the injector is placed in the reaction vessel, the end thereof may be either immersed in the reaction solution or placed above the solution. A tail pipe may be provided below the injector and immersed in the solution.

If the process of the present invention is not employed, it is impossible to obtain saccharide oxides having an excellent hue in a high yield with a high selectivity by oxidizing the saccharides with molecular oxygen in a short period of time. The following examples further illustrate the invention in more detail. In the examples, percentages are given by weight.

# 0 048 974

### Example 1

9.5 g of active carbon was suspended in 100 ml of water. Palladium chloride containing 0.5 g of metallic palladium was added to the suspension to adsorb palladium chloride on the active carbon at room temperature. Then, 7 ml of 20% aqueous sodium hydroxide solution and 1 ml of 37% aqueous formalin solution were added thereto. After the treatment at 80°C for one hour followed by the filtration, washing with water and drying, 5% palladium carbon catalyst was obtained.

Then, an aqueous glucose solution was subjected to the oxidation reaction in a reaction device which comprised a vessel 1 having an inner diameter of about 13 cm and a height of about 100 cm and having a liquid-circulating pipe 3 through a circulating pump 2, the pipe 3 being provided with an injector 4 at an end thereof placed in the vessel 1 as shown in Fig. 1. More concretely, 3.3 Kg of 30% aqueous glucose solution and 5.0 g (0.5% based on glucose) of 5% palladium carbon catalyst were charged in the vessel 1. Thus obtained catalyst suspension was circulated through the circulating pump 2, circulating pipe 3 and injector 4. A space 5 in the vessel is connected with an atmospheric pressure oxygen-supplying device via a nozzle 6 and always filled with atmospheric pressure oxygen. An outlet of the injector 4 is immersed in the suspension. A gas inlet 7 of the injector 4 is open to the space 5. In this case, the liquid was circulated at a rate of 12 liters per minute and amount of oxygen sucked by means of the injector was 15 liters per minute.

As the reaction proceeded, 40% aqueous sodium hydroxide solution was added dropwise to the liquid through at inlet 9 to keep the liquid at pH 9.5—10. The reaction temperature was maintained at 50±1°C. After the initiation of the circulation, the sampling was effected at intervals to determine a conversion of glucose. The conversion was determined by subjecting the sample from which the catalyst had been filtered out to the reduced pressure evaporation to dryness, converting the same to its trimethylsilyl derivative with a compound which introduce the trimethylsilyl group therein, subjecting the product to gas chromatography and calculating a value of the conversion from the area ratio thereof (%). The reaction was completed in about 110 minutes. The results are shown in Table 1. Upon completion of the reaction, hue of the product and rates of the by-products formed and unreacted compound were also examined.

The amount of the by-products were calculated from an area ratio of saccharic acid to impurities in the gas chromatography.

TABLE 1

| Time (mins.) | 0 | 20 | 50 | 100 | 110 |
|---|---|---|---|---|---|
| Conversion (%) | 0 | 31 | 59 | 95 | 97 |
| Hue (Gardner) | — | — | — | — | 4—5 |
| By-products (%) | — | — | — | — | 2.1 |

It is understood from the results shown in Table 1 that gluconic acid can be obtained with a conversion of at least 95% after the reaction carried out for about 100—110 minutes.

### Example 2

The oxidation reaction of glucose was carried out using the same catalyst and the same device as in Example 1 but with varied amounts of the catalyst and reaction temperature. pH of the reaction liquid was 9.5—10 and the circulating rate of the liquid was 12 liters per minute. The results are shown in Table 2.

4

# 0 048 974

TABLE 2

| Reaction conditions | Experiment No. | | | | |
|---|---|---|---|---|---|
| | 1 | 2 | 3 | 4 | 5 |
| Amount of glucose (Kg) | 1.35 | 1.35 | 1.35 | 1.35 | 0.90 |
| Amount of aqueous glucose solution (Kg) | 4.50 | 4.50 | 4.50 | 4.50 | 4.50 |
| Reaction temperature (°C) | 50 | 50 | 45 | 55 | 50 |
| 5% Pd-carbon catalyst (g) | 6.75 | 13.0 | 6.75 | 6.75 | 6.75 |
| Reaction time (mins.) | 95 | 60 | 160 | 70 | 120 |
| Conversion (%) | 98 | 98 | 97 | 98 | 97 |
| Hue (Gardner) | 5 | 4 | 4 | 6 | 4 |
| By-products (%) | 1.5 | 0.7 | 0.9 | 2.8 | 1.7 |

Example 3

A 300 l reaction vessel of the type as shown in Fig. 1 was used. 100 Kg of 30% aqueous glucose solution was charged in the reaction vessel. 0.5%, based on glucose, of commercially available palladium carbon catalyst was added thereto. The glucose oxidation reaction was carried out under conditions comprising a temperature of 50°C, pH of 9.5—10, liquid circulation rate of 170 l/min and oxygen gas introducing rate of 620 l/min. The results are shown in Table 3.

TABLE 3

| Time (mins.) | 0 | 30 | 60 | 90 | 120 |
|---|---|---|---|---|---|
| Conversion (%) | 0 | 30 | 55 | 84 | 97 |
| Hue (Gardner) | — | — | — | — | 5 |
| By-products (%) | — | — | — | — | 2.3 |

Comparative Example 1

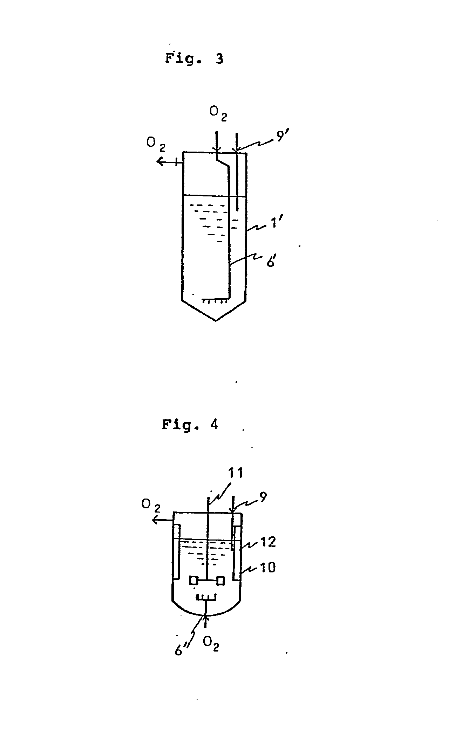

The oxidation reaction was carried out using a reaction device 1' having an inner diameter of about 13 cm, a height of about 100 cm and a gas inlet 6' at the bottom thereof as shown in Fig. 3 in place of the reaction device shown in Fig. 1. During the reaction, oxygen was introduced through the inlet 6' in the form of fine bubbles and 40% aqueous NaOH solution was fed therein so as to maintain the pH at 9.5—10. Time, glucose conversion, hue and amount of by-products were determined in the same manner as in Example 1. The results are shown in Table 4.

The reaction temperature was maintained at 50±2°C, amount of the liquid charged was 6.4 Kg, the catalyst concentration was 1.0 wt.%, based on glucose, of palladium carbon catalyst containing 0.5% palladium, and oxygen introducing rate was 990 l/min.

TABLE 4

| Time (mins.) | 0 | 50 | 100 | 200 | 300 | 430 |
|---|---|---|---|---|---|---|
| Conversion (%) | 0 | 9.5 | 20 | 29 | 41 | 61 |
| Hue (Gardner) | — | — | — | — | — | 10 |
| By-products (%) | — | — | — | — | — | 10 |

5

Comparative Example 2

A reaction device as shown in Fig. 4 was used which comprised a cylindrical vessel 10 having an inner diameter of about 120 cm and a height of about 30 cm wherein baffles were provided on the side wall and a stirrer 11 was suspended. An oxygen inlet 6'' was provided at the bottom of the vessel. 2.6 Kg of 30 wt.% aqueous glucose solution in which the same concentration of the catalyst had been suspended was charged therein. The reaction was carried out at a temperature maintained at 50±2°C and at a stirring rate of 500 rpm while oxygen was introduced therein in the form of fine bubbles at a rate of 394 l/min through the inlet 6''. The liquid was maintained at a pH of 9.5—10.0. The results are shown in Table 5.

TABLE 5

| Time (mins.) | 0 | 50 | 100 | 200 | 300 | 520 |
|---|---|---|---|---|---|---|
| Conversion (%) | 0 | 15 | 24 | 42 | 59 | 90 |
| Hue (Gardner) | — | — | — | — | — | 10—11 |
| By-products (%) | — | — | — | — | — | 6 |

4. Brief description of drawings

Fig. 1 is a schematic front view of a reaction device used in the process of the present invention. Fig. 2 is a cross section showing an embodiment of injector used in the present invention. Figs. 3 and 4 are schematic front views of reaction devices used in Comparative Examples 1 and 2, respectively.

1 . . . Vessel, 2 . . . Circulating pump, 3 . . . Circulating pipe, 4 . . . Injector, 6 . . . Oxygen-supply nozzle, 7 . . . Gas inlet.

**Claim**

A process for oxidizing a water-soluble saccharide to obtain the corresponding carboxylic acid derivative, which comprises: in a reaction vessel, contacting a gas containing molecular oxygen with a bath of a liquid reaction mixture of an aqueous solution of said saccharide having suspended therein particles of a palladium catalyst supported on carbon, at a temperature in the range of 30° to 60°C and a pH maintained in the range of 8 to 11, whereby to obtain said carboxylic acid derivative, characterized in continuously withdrawing a portion of said reaction mixture from said reaction vessel, feeding same through a pipe located outside of said vessel by means of a circulating pump, then feeding the reaction mixture from said pipe into and through a mixing injector, inducing a flow of said gas from a source of supply thereof into said mixing injector caused by the velocity of flow of said reaction mixture through said mixing injector and therein mixing said gas with said reaction mixture flowing through said mixing injector, and then feeding the mixture of said gas and said reaction mixture discharged from said mixing injector into said bath of said reaction mixture in said reaction vessel below the upper surface thereof, and wherein the ratio V2/V1 is in the range of from 0,1 to 10,0 and the ratio V3/V2 is in the range of 0.1 to 100.0, wherein V1 is the volume, in liters, of said bath in said vessel, V2 is the volumetric rate of flow, in liters per minute, of the reaction mixture circulating through said pipe and V3 is the volume, in liters per minute, of oxygen mixed with said reaction mixture in said mixing injector.

**Patentanspruch**

Verfahren zur Oxidierung eines wasserlöslichen Saccharides zur Bildung des entsprechenden Carboxylsäurederivaten, wobei man in einem Reaktionsgefäß ein Gas, welches molekularen Sauerstoff enthält, mit einem Bad einer flüssigen Reaktionsmischung aus einer wässrigen Lösung des Saccharids, in welchem Partikel eines Palladiumkatalysators auf einem Kohlenstoffträger suspendiert sind, bei einer Temperatur im Bereich von 30°—60°C und einem pH-Wert im Bereich von 8—11 in Kontakt bringt, wodurch man das Carboxylsäurederivat erhält, dadurch gekennzeichnet, daß man kontinuierlich einen Teil der Reaktionsmischung aus dem Reaktionsgefäß abzieht, mit Hilfe einer Zirkulationspumpe die Mischung durch ein Rohr führt, das sich außerhalb des Reaktionsgefäßes befindet, dann die Reaktionsmischung aus dem Rohr durch einen Mischinjektor leitet, in welchen man den Gasstrom aus einem Versorgungsanschluß einführt, mittels der Strömungsgeschwindigkeit der Reaktionsmischung durch den Mischinjektor, in welchem das Gas mit der durchströmenden Reaktionsmischung vermischt wird, worauf man die den Mishinjektor verlassende Mischung aus dem Gas und der Reaktionsmischung in das Bad der Reaktionsmischung innerhalb des Reaktionsgefäßes unterhalb der Oberfläche ein führt, wobei das Verhältnis V2/V1 im Bereich von 0,1 bis 10,0 und das Verhältnis V3/V2 im Bereich von 0,1 bis 100,0 liegt, wobei V1 das Volumen des Bades in dem Gefäß in Litern ist, V2 das volumetrische Strömungsausmaß der Reaktionsmischung, die durch das rohr geführt wird, in Litern pro Minute darstellt und V3 das Volumen des Sauerstoffes in Litern pro Minute ist, der innerhalb des Mischinjektors mit der Reaktionsmischung vermischt wird.

# 0 048 974

**Revendication**

Procédé d'oxydation d'un saccharide soluble dans l'eau un vue d'obtenir le dérivé acide carboxylique correspondant, procédé dans lequel on met en contact dans un récipient de réaction un gaz contenant de l'oxygène moléculaire avec un bain de mélange réactionnel liquide d'une solution aqueuse de saccharide ayant en suspension des particules de catalyseur au palladium sur support de carbone, à une température dans la plage de 30° à 60°C et à un pH maintenu dans la plage de 8 à 11, afin d'obtenir le dérivé acide carboxylique, caractérisé en ce que l'on retire de manière continue une partie du mélange réactionnel du récipient de réaction, on envoie ce mélange par un conduit situé à l'extérieur du récipient à l'aide d'une pompe à circulation, puis on envoie le mélange réactionnel du conduit dans et à travers un injecteur-mélangeur, on induit un courant du gaz à partir d'une source d'approvisionnement dans l'injecteur-mélangeur par action de la vitesse d'écoulement du mélange réactionnel à travers l'injecteur-mélangeur et on y mélange le gaz avec le mélange réactionnel s'écoulant à travers l'injecteur-mélangeur, et ensuite on introduit le mélange de gaz et de mélange réactionnel, s'écoulant de l'injecteur-mélangeur dans le bain de mélange réactionnel, dans le récipient de réaction au dessous de sa surface supérieure, et que le rapport V2/V1 est dans la plage de 0,1 à 10,0 et le rapport V3/V2 est dans le rapport 0,1 à 100,0, V1 étant le volume, en litres, du bain dans le récipient, V2 étant le débit volumétrique, en litres par minute, du mélange réactionnel circulant à travers le conduit et V3 étant le volume, en litres par minute, de l'oxygène mélangé avec le mélange réactionnel dans l'injecteur-mélangeur.

Fig. 1

Fig. 2

Fig. 3

Fig. 4